# EUROPEAN PATENT APPLICATION

(11) **EP 1 574 568 A1**
(43) Date of publication of application: **14.09.2005**
(21) Application number: 03780848.2
(22) Date of filing: 17.12.2003
(51) Int. Cl.: C12N 9/80, C12N 1/20, C12P 41/00

(54) **D-AMINOACYLASE**

(30) Priority: 18.12.2002 JP 2002366389; 10.10.2003 JP 2003351560
(71) Applicant: DAIICHI PURE CHEMICALS CO., LTD., Tokyo 103-0027 (JP); Isobe, Kimiyasu, Iwate 020-0111 (JP)
(72) Inventor: ISOBE, Kimiyasu, Morioka-shi, Iwate 020-0111 (JP); YAMAGUCHI, Seiki, Daiichi Pure Chemicals Co., Ltd., Iwate-gun, Iwate 028-7305 (JP); KOBAYASHI, Masayuki, Daiichi Pure Chemicals Co Ltd, Iwate-gun, Iwate 028-7305 (JP); KUMAGAI, Shinya, Daiichi Pure Chemicals Co., Ltd., Iwate-gun, Iwate 028-7305 (JP); SARASHINA, Takami, Daiichi Pure Chemicals Co., Ltd, Naka-gun, Ibaraki 319-1112 (JP)
(74) Representative: Hartz, Nikolai
(86) International application number: PCT/JP2003/016182
(87) International publication number: WO 2004/055179

(57) **Abstract**

A D-aminoacylase having a high substrate specificity is provided. This D-aminoacylase can produce D-amino acids from N-acetyl-D,L-amino acids conveniently and efficiently at a low cost.

A D-aminoacylase produced by a microorganism of genus Defluvibacter; which acts on a N-acetyl-D-amino acid; which has a molecular weight (as determined by electrophoresis) of about 55,000 daltons, and an isoelectric point (as determined by two-dimensional electrophoresis for denatured system) of 5.3; which acts on N-acetyl-D-valine, N-acetyl-D-leucine, and the like, but not on N-acetyl-L-valine, N-acetyl-L-leucine, and the like; which has an optimal temperature of 37°C (pH 8) and an optimal pH value of 8 to 8.5 at 37°C; and whose activity is inhibited by Mn²⁺, Co²⁺, Ni²⁺, and Zn²⁺ each at 1 mmol/L, and by dithiothreitol, 2-mercaptoethanol, o-phenanthroline, and L-cysteine each at 5 mmol/L.

## Description

### TECHNICAL FIELD

This invention relates to a novel D-aminoacylase produced by a bacterium belonging to genus Defluvibacter, and a method for producing a D-amino acid adapted for use in drugs and chemical products by using the D-aminoacylase.

### BACKGROUND ART

D-amino acids are lately found to be an effective ingredient as a raw material of drugs and other pharmaceuticals, and production of the D-amino acids of high optical purity at low cost has become an issue of significance in the industry. The approach generally employed is resolution of a chemically synthesized racemate, and enzymatic production has drawn attention since such process is free from generation of byproducts and a large amount of waste solvents.

One known enzymatic process for producing the D-amino acid is the process wherein action of a D-aminoacylase on a N-acetyl-D,L-amino acid is utilized for the specific production of the D-amino acid, and this method has been commercially adopted in producing the D-amino acid.

Microorganisms which produce a D-aminoacylase include Pseudomonas sp. AAA6029 strain (for example, Chemical and Pharmaceutical Bulletin (US), 1978, vol.26, p. 2698), Streptomyces olivaceus S·62 strain (for example, Japanese Patent Application Laid-Open No. Sho 53-59092), and Alcaligenes xylosoxydans subsp. xylosoxydans A-6 strain (for example, Japanese Patent Application Laid-Open No. Hei 2-234677), and D-aminoacylases produced by these microorganisms have also been reported.

These D-aminoacylases, however, exhibit markedly different reaction characteristics depending on the type of the N-acetyl-D,L-amino acid, and it has been difficult to produce a wide variety of D-amino acids at low cost by using such known D-aminoacylases.

Commercial production of the D-amino acid by using a genetically engineered D-aminoacylase has also been disclosed (for example, Japanese Patent Application Laid-Open No. 2001-185 and Japanese Patent Application Laid-Open No. 2001-275688). However, a large amount of enzyme would be required for the production of a D-amino acid from the N-acetyl-D-amino acid which essentially has a low reactivity, and this poses a limitation on the cost and amount of the production.

### DISCLOSURE OF THE INVENTION

In the present invention, a novel microorganism which produces a D-aminoacylase having a high activity for the N-acetyl-D-amino acid for which conventionally reported enzymes had low activity was found in nature to thereby provide a novel method for producing a D-aminoacylase capable of producing a D-amino acid at a low cost, as well as a method for producing a D-amino acid utilizing such novel D-aminoacylase. Also provided is a microorganism which produces such novel D-aminoacylase.

The inventors of the present invention have made an intensive study to solve the problems as described above, and found in nature a bacterial species belonging to genus Defluvibacter which exhibits a high activity for the substrates for which conventional enzymes had only limited reactivity, and the present invention has been completed on the bases of such finding.

More specifically, this invention provides a D-aminoacylase which has the following enzymological characteristics.
(a) action: acting on a N-acetyl-D-amino acid to produce a D-amino acid;
(b) molecular weight: about 55,000 daltons when determined by SDS-polyacrylamide gel electrophoresis;
(c) isoelectric point: an isoelectric point of 5.3 when measured by two-dimensional electrophoresis for denatured system;
(d) substrate specificity: acting on N-acetyl-D-amino acids, and in particular, on N-acetyl-D-valine, but not on N-acetyl-L-amino acids (acting on substrates including N-acetyl-D-valine, N-acetyl-D-leucine, N-acetyl-D-methionine, N-acetyl-D-triptophan, N-acetyl-D-phenylalanine, and N-acetyl-D-tyrosine, but not on N-acetyl-L-valine, N-acetyl-L-leucine, N-acetyl-L-methionine, N-acetyl-L-tryptophan, N-acetyl-L-phenylalanine, and N-acetyl-L-tyrosine);
(e) thermostability: relatively stable at 4°C to 30°C when heated at pH 8.5 for 1 day;
(f) optimal temperature: optimally active at 37°C when reacted at pH 8 for 30 minutes;
(g) pH stability: stable near pH 9, and relatively stable near pH 7 to 10 when heated at a temperature of 30°C for 1 day;
(h) optimal pH: optimally active near pH 8 to 8.5 when reacted at 37°C;
(i) effects of metal ions: activity is inhibited by Mn²⁺, Co²⁺, Ni²⁺ , and Zn²⁺ each at 1 mmol/L; and
(j) effects of inhibitors: activity is inhibited by dithiothreitol, 2-mercaptoethanol, o-phenanthroline, and L-cysteine each at 5 mmol/L.

This invention also provides a D-aminoacylase comprising a protein defined in either one of the following (a) and (b) as well as a gene coding such D-aminoacylase.
(a) a protein comprising the amino acid sequence of SEQ ID NO.2; and
(b) a protein comprising an amino acid sequence of SEQ ID NO.2 wherein substitution, deletion, or addition of one to several amino acids has occurred , and having D-aminoacylase activity.

This invention also provides a microorganism of genus Defluvibacter which products a D-aminoacylase that efficiently converts a N-acetyl-D,L-amino acid or a N-acetyl-D-amino acid to a D-amino acid.

This invention also provides a method for producing the D-aminoacylase wherein the microorganism is cultivated, and the D-aminoacylase is recovered from the culture.

This invention also provides a method for producing a D-amino acid wherein the D-aminoacylase acts on a N-acetyl-D,L-amino acid or a N-acetyl-D-amino acid.

The novel D-aminoacylase produced by the microorganism of genus Defluvibacter according to the present invention has a high substrate specificity, and it can produce D-amino acids from, for example, N-acetyl-D,L-valine, N-acetyl-D,L-methionine, N-acetyl-D,L-tryptophan, N-acetyl-D,L-leucine, N-acetyl-D,L-phenylalanine, and N-acetyl-D,L-tyrosine conveniently and efficiently at a low cost.

### BRIEF DESCRIPTION OF DRAWINGS

FIG. 1 is a view showing an electrophoretogram of the enzyme of the present invention that was taken in the course of molecular weight measurement by the electrophoresis.
FIG. 2 is a graph showing the residual activity that was measured in the course of evaluating thermostability of the enzyme of the present invention.
FIG. 3 is a graph showing the relative activity that was measured in the course of evaluating optimal temperature of the enzyme of the present invention.
FIG. 4 is a graph showing the residual activity that was measured in the course of evaluating pH stability of the enzyme of the present invention.
FIG. 5 is a graph showing the relative activity that was measured in the course of evaluating optimal pH of the enzyme of the present invention.
FIG. 6 is a graph showing resolution rate of N-acetyl-D,L-valine.

### BEST MODE FOR CARRYING OUT THE INVENTION

The present invention was completed after finding a microorganism which is capable of producing a novel D-aminoacylase, characterizing the novel D-aminoacylase and finding the gene therefor, and demonstrating that the novel D-aminoacylase is effective for producing the D-amino acid.

More specifically, while the microorganism which produces the novel D-aminoacylase of the present invention is not limited to any particular species as long as it produces the D-aminoacylase of the present invention, a typical novel D-aminoacylase-producing microrganism found in the present invention is the microorganism of genus Defluvibacter isolated from the soil of Daiichi Pure Chemicals Co., Ltd., Iwate factory. An example of the microorganism is Defluvibacter sp. A131-3. This A131-3 strain has the following bacteriological characteristics. (Morphological finding)
1. Cell morphology: bacillus (0.6 to 0.7 x 1.5 to 2. 0 µm)
2. Gram staining: negative
3. Spore formation: no
4. Motility: yes
5. Flagella: yes
6. Normal agar medium: circle, smooth edge, slightly concave, shiny, subdued gray to pale yellow

### (Physiological property)

1. Catalase production: positive
2. Oxidase production: positive
3. Acid/gas production (glucose): negative
4. O/F test(glucose): negative
5. Anaerobic growth: no
6. Aerobic growth: absolutely aerobic

### (Biological property)

Biochemical test was conducted by using API20NE identification system (bioMerieux, France) in accordance with its instruction.
1. Nitrate reduction: negative
2. Indol production: negative
3. Glucose acidification: negative
4. Arginine dihydrase: negative
5. Urease: negative
6. Esculin hydrolysis: negative
7. Gelatin hydrolysis: negative
8. β-galactosidase: negative
9. Cytochrome oxidase: positive

### (Utilization test)

1. Glucose: positive
2. L-arabinose: negative
3. D-mannose: positive
4. D-mannitol: negative
5. N-acetyl-D-glucosamine: positive
6. Maltose: negative
7. Potassium gluconate: positive
8. N-capric acid: negative
9. Adipic acid: negative
10. DL-malic acid: positive
11. Sodium citrate: negative
12. Phenyl acetate: negative
13. 2,4-dichlorophenol: negative
14. Phenol: negative

### (Analysis of fatty acid composition)

composition of the fatty acid was measured by using gas chromatographic system HP6890 (Hewlett-Packard, CA, USA). Bacterial species data was compared by using Sherlock Microbial Identification System (MIDI, DE, USA). The database used was TSBA (Version 4.0) from MIS Standard Libraries (MIDI, DE, USA).
1. Major fatty acid: C_{18:1}ω7c straight chain monounsaturated fatty acid
2. Hydroxy fatty acid: C_{12:0}3OH

### (Ubiquinone analysis)

Molecular species was identified using high performance liquid chromatography by comparing the retention time with that of the ubiquinone standard specimen.

### 1. Major ubiquinone system: Q-10

### (Analysis of cell wall amino acid)

Specific amino acid was detected by using high performance thin layer chromatography (HPTLC) plate (Merck, NJ, USA) and developing the specific amino acid contained in the cell wall peptide-glucan as a control.

Cell wall amino acid: meso-diaminopimelic acid

### (Analysis of 16SrDNA-Full base sequence)

Homology search was performed using BLAST for DNA base sequence database (GenBank).

### 1. 16SrDNA homology of 99.9% with Defluvibacter lusatiensis DSM11099)

The microorganism newly discovered in nature was classified as a bacterium belonging to genus Defluvibacter from these biochemical and bacteriological characteristics. A microorganism having similar characteristics, namely, Defluvibacter lusatiensis DSM11099 of genus Defluvibacter has already been reported (Defluvibacter lusatiae gen. nov., sp. nov., a new chlorophenol-degrading member of the α-2 subgroup of proteobacteria. Syst. Appl. Microbiol., 1999, 22, 197-204). However, there is no description that this known bacterium of genus Defluvibacter produces the D-aminoacylase, and the present invention has for the first time revealed that this microorganism of genus
Defluvibacter has the ability of producing D-aminoacylase. It is to be noted that the bacterial strain found in the present invention was designated as Defluvibacter sp. A131-3, and deposited on September 26, 2002 to The National Institute of Advanced Industrial Science and Technology (an Independent Administrative Institution), International Patent Organism Depositary (Chuo-6, 1-1, Higashi 1-chome, Tsukuba-shi, Ibaraki, Japan (Post code, 305-8566)) as FERM BP-08563.

It is also to be noted that the novel D-aminoacylase found in the present invention may also be produced by using a strain produced by genetic engineering, alteration, or modification from the parent strain of the bacterium of the genus Defluvibacter, for example, Defluvibacter sp. A131-3 by utilizing deliberate or random mutation, or known recombinant DNA manipulation technique which is commonly used to improve productivity or nature of an enzyme.

The novel D-aminoacylase of the present invention can be obtained by inoculating the microorganism in an appropriate medium followed by cultivation.

The medium used in the cultivation may be any medium commonly used for the cultivation of a microorganism as long as the microorganism of interest can grow and produce the novel D-aminoacylase. The medium, however, preferably contains an appropriate amount of nitrogen source, carbon source, and inorganic salts which are utilizable by the microorganism.

The nitrogen source, the carbon source, and the inorganic salts are not particularly limited.

Exemplary nitrogen sources include meat extract, yeast extract, and peptone. Exemplary carbon sources include glucose, fructose, sucrose, glycerin, and acetic acid. Exemplary inorganic salts include disodium hydrogen phosphate, potassium dihydrogen phosphate, magnesium sulfate, ammonium nitrate, iron sulfate, and zinc sulfate.

The D-aminoacylase of the present invention does not require any inducer for its production. However, in order to produce the D-aminoacylase at a high rate, a N-acetyl-D-amino acid, N-acetyl-D,L-amino acid, or other acylated amino acid derivative is preferably added as an inducer to the medium in an amount of about 0.01 to 0.5% by weight (hereinafter simply referred to as "%"), and N-acetyl-D-valine, N-acetyl-D-leucine, and the like are particularly effective inducers.

The culture is not limited for its pH range as long as it admits bacterial growth, although the pH is preferably in the range of about 7 to 9. The temperature of the culture is preferably kept at 15 to 40°C, and more preferably, at 25 to 37°C. The cultivation is preferably continued for 20 to 48 hours using a liquid medium with shaking. The time, however, may vary depending on the medium used. The cultivation of the bacterium may also take place in a solid medium having agar added to the medium similar to the one as described above.

The D-aminoacylase is produced within the bacterial cell of the microorganism obtained by the process as described above.

Recovery and purification of the target D-aminoacylase from the culture may be performed according to the process used for the recovery and purification of common enzymes, namely, by separating the microorganism by centrifugation, filtration, or the like, lysing the cell by mechanical grinding, ultrasonication, or the like, and recovering and purifying the enzyme from the cell lysate by common separation means such as hydrophobic chromatography, ion-exchange chromatography, hydroxyapatite chromatography, gel filtration chromatography, or the like.

The D-aminoacylase of the present invention thus obtained had the enzymological characteristics and the amino acid sequence as described below. The base sequence of the gene for the D-aminoacylase of the present invention is also shown below.
(1) Action: The D-aminoacylase acts on a N-acetyl-D-amino acid to produce a D-amino acid.
(2) Molecular weight: Molecular weight is measured by SDS-polyacrylamide gel electrophoresis (using PAG Mini "Daiichi" 10/20 manufactured by Daiichi Pure Chemicals Co.,Ltd.) according to the process commonly used in the art, and the molecular weight determined from the mobility of protein molecular weight markers (manufactured by Daiichi Pure Chemicals Co.,Ltd., protein molecular weight markers "Daiichi"·III) is about 55,000 daltons.
(3) Isoelectric point: Isoelectric point is measured on the basis of two-dimensional electrophoresis process by two-dimensional electrophoresis for denatured system (using IPG tube gel "Daiichi" 4-10 and PAG Large "Daiichi" 2D-10/20 manufactured by Daiichi Pure Chemicals Co.,Ltd.). The pI value of 5.3 is calculated from mobility of 2D-protein isoelectric point markers (2D-protein isoelectric point markers "Daiichi" manufactured by Daiichi Pure Chemicals Co.,Ltd.).
(4) Substrate specificity: Substrate specificity of the acylase of the present invention was confirmed by using the following N-acetyl-D-amino acids and N-acetyl-L-amino acids as the substrate and combining therewith the D-amino acid oxidase or the L-amino acid oxidase. The acylase of the present invention acts on the following N-acetyl-D-amino acids but not on the N-acetyl-L-amino acids. Of the N-acetyl-D-amino acids, the acylase of the present invention acts most effectively on N-acetyl-D-valine, and to some extent on N-acetyl-D-leucine, N-acetyl-D-methionine, N-acetyl-D-tryptophan, N-acetyl-D-phenylalanine, and N-acetyl-D-tyrosine. The acylase of the present invention does not act on N-acetyl-L-valine, N-acetyl-L-leucine, N-acetyl-L-methionine, N-acetyl-L-tryptophan, N-acetyl-L-phenylalanine, and N-acetyl-L-tyrosine.
   In the measurement of the reactivity with the L-amino acids, an oxidase for L-amino acids is used instead of the D-amino acid oxidase in the activity measurement as will be described below.
(5) Thermostability: Thermostability is measured by heating the solution of the acylase of the present invention to 4°C, 25°C, 30°C, 40°C, and 50°C for 1 day at pH 8.5 and measuring the residual enzymatic activity by the activity measurement method as will be described below. The enzymatic activity is relatively stable at 4°C to 30°C.
(6) Optimal temperature: Enzymatic activity is measured at pH 8 and at 4°C, 25°C, 30°C, 37°C, and 40°C by the activity measurement method as will be described below. It is then found that the activity is optimal at 37°C.
(7) pH stability: By heating to a temperature of 30°C for 1 day at pH in the range of 4 to 12 and measuring the residual enzymatic activity by the activity measurement method as will be described below, it is found that the acylase of the present invention is most stable near pH 9, and relatively stable near pH 7 to near pH 10.
(8) Optimal pH: By heating to a temperature of 37°C at pH in the range of 6 to 12 and measuring the enzymatic activity by the activity measurement method as will be described below, it is found that the acylase of the present invention is most active near pH 8 to 8.5.
(9) Effects of metal ion: To the enzyme solution is added a metal ion, namely, calcium chloride dihydrate, iron (III) chloride hexahydrate, sodium chloride, cobalt (II) chloride hexahydrate, potassium chloride, nickel chloride hexahydrate, magnesium chloride hexahydrate, copper (II) sulfate pentahydrate, manganese (II) chloride tetrahydrate, zinc chloride, or sodium molybdate in an amount of 1 mmol/L, and the solution is reacted with N-acetyl-D,L-valine to measure the amount of the D-valine produced by HPLC. It is then found that the activity is inhibited by Mn²⁺, Co²⁺, Ni²⁺, and Zn²⁺ each at 1 mmol/L.
(10) Effects of inhibitor: To the enzyme solution is added an inhibitor, namely, ethylenediamine tetraacetic acid, 2-mercaptoethanol, N-ethylmaleimide, o-phenanthroline, L-cysteine, iodacetamide, or dithiothreitol to 5 mmol/L, and the solution was reacted with N-acetyl-D,L-valine to measure the amount of the D-valine produced by HPLC. It is then found that the activity is inhibited by dithiothreitol, 2-mercaptoethanol, o-phenanthroline, and L-cysteine each at 5 mmol/L.

Base sequence of the D-aminoacylase gene and amino acid sequence of the D-aminoacylase protein of the present invention were determined by the method known in the art as described below.

PCR was conducted by using a primer mix including all of the DNA sequences that are estimated from the N-terminal and the internal amino acid sequences of the purified enzyme, and the DNA that had been extracted and purified from the Defluvibacter sp. A131-3; and then the resulting amplification product was cloned. The base sequence of the D-aminoacylase gene of the present invention was then determined to be the base sequence of SEQ ID NO. 1. It was also found from this base sequence that the D-aminoacylase protein of the present invention has the amino acid sequence of SEQ ID NO.2.

The D-aminoacylase of the present invention may comprise not only (a) a protein comprising the amino acid sequence of SEQ ID NO.2, but also (b) a protein comprising an amino acid sequence of SEQ ID NO.2 wherein substitution, deletion, or addition of one to several amino acids has occurred, and having D-aminoacylase activity. The amino acid sequence wherein substitution, deletion, or addition of one to several amino acids has occurred in the amino acid sequence includes an amino acid sequence which has homology of at least 80%, preferably at least 90%, and more preferably at least 95% with the amino acid sequence of the SEQ ID NO. 2.

The D-aminoacylase gene of the present invention may be any gene as long as it codes for the protein (a) or (b), and the D-aminoacylase gene preferably comprises not only (c) a DNA comprising the base sequence defined by SEQ ID NO. 1 but also (d) a DNA which hybridizes under stringent conditions with the DNA comprising the base sequence which is complimentary to the base sequence defined by SEQ ID NO. 1, and which codes for a protein having D-aminoacylase activity. The typical stringent conditions include conditions wherein the hybridization takes place in 0.2x SSC containing 0.1% SDS at 50°C, or in 1x SSC containing 0.1% SDS at 60°C. The DNA which hybridizes under the above stringent conditions includes a DNA which has a homology of at least 80%, preferably at least 90%, and more preferably at least 95% with the base sequence defined by SEQ ID NO. 1.

Measurement of acylase activity using amino acid oxidase: In 10 mL of 0.1 mol/L phosphate buffer solution (pH 8) were dissolved 0.61 mg of 4-aminoantipyrine (manufactured by Nacalai Tesque, Inc., Code: 01907-52), 3.22 mg of N-ethyl-N-(2-hydroxy-3-sulfopropyl)-3-methlaniline, sodium salt, dihydrate (manufactured by Dojindo Laboratories, Code: OC13), 30 units of peroxidase (manufactured by SIGMA Corp., Code: P-6782), and 1 unit of D-amino acid oxidase (manufactured by SIGMA Corp., Code: A-9128) or 1 unit of L-amino acid oxidase (manufactured by SIGMA Corp., Code: A-5147) to prepare a chromogenic reagent. A 1 mL of the reaction solution containing 500 µL of this chromogenic reagent, 100 µL of 100 mmol/L N-acetyl-D,L-valine, 100 µL of the target enzyme sample, and 300 µL of 0.1 mol/L phosphate buffer solution (pH 8) was heated at 37°C for 30 minutes, and subsequently the solution was measured for its absorbance at 555 nm by a spectrophotometer to determine the enzymatic activity by referring to the calibration curve that had been prepared by using D-valine.

It is to be noted that 1 U is the amount of the enzyme that catalyzes generation of 1 µmol of D-valine in 1 minute.

### Measurement of acylase using HPLC:

The analysis was carried out by using Inertsil ODS-2 column (manufactured by GL Science Inc.) and a buffer solution containing 0.015% 1-sodium pentanesulfonate (pH 2.5) and acetonitrile in a ratio of 80 : 20 at a flow rate of 0.5 mL/minute. The detection was conducted at 230 nm. The column was analyzed at a temperature of 30°C. The enzymatic activity was evaluated in terms of the ratio between the area of the acetyl compound and the free compound, and the resolution rate was expressed as a relative activity in relation to the resolution rate of 100 in the case when no acylase was added.

The resulting novel D-aminoacylase specifically acts on N-acetyl-D-amino acids but not on N-acetyl-L-amino acids, and therefore, it can be used for producing a D-amino acid from an N-acetyl-D-amino acid. It can also be used in the resolution, therefore, in the separation of D-amino acids from N-acetyl-D,L-amino acids. Separation of a D-amino acid from an L-amino acid can be accomplished by acetylating a D,L-amino acid to produce the corresponding N-acetyl-D,L-amino acid, and then adding thereto the D-aminoacylase to hydrolyze the N-acetyl-D-amino acid to produce the D-amino acid. It is to be noted that, if only an N-acetyl-D-amino acid is used, the resulting compound will solely yield a D-amino acid.

When D-aminoacylase is to be acted on an N-acetyl-D,L-amino acid or N-acetyl-D-amino acid, the D-aminoacylase is generally added to the aqueous solution of the substrate in an amount of 1 to 1000 U/mL, and preferably in an amount of 50 to 500 U/mL. The N-acetyl-D,L-amino acid or the N-acetyl-D-amino acid is preferably provided in the form of an aqueous solution of 1 to 40 % by weight (hereinafter referred to as "%"), and more preferably, in the form of a 5 to 25% aqueous solution.

The reaction temperature is preferably 10 to 50°C, and more preferably 15 to 45°C, and the reaction is preferably conducted at a pH of 6.5 to 10.5, and more preferably at 7.5 to 10. The reaction time is preferably 0.2 to 10 days, and more preferably 1 to 5 days.

The D-amino acid may be separated and recovered from the reaction solution by utilizing a known means such as concentration, isoelectric point, precipitation, treatment using an ion exchange resin, and membrane separation.

### EXAMPLES

Next; the present invention is described in further detail by referring to the Examples which by no means limit the scope of the present invention.

### Example 1: Isolation of Defluvibacter sp. A131-3 strain

Soil was collected at Daiichi Pure Chemicals Co., Ltd., Iwate factory, and the collected soil was treated as described below to recover the bacterial cells.

A small amount of the soil was added to a culture medium at pH 8.5 containing 0.2% ammonium nitrate, 0.2% potassium dihydrogen phosphate, 0.1% disodium hydrogen phosphate, 0.05% magnesium sulfate heptahydrate, and 0.2% N-acetyl-D,L-valine (an inducer), and the medium was incubated at 30°C in a test tube with shaking. Next, a culture solution was plated out (inoculated) to a plate culture medium of the same composition additionally containing 2% agar, and the plate culture medium was incubated at 30°C to separate the microorganisms that had grown in the medium.

The thus separated microorganisms were again cultivated in the culture medium of the same composition in a test tube with shaking to select a microorganism which has a D-aminoacylase-producing ability different from conventional microorganisms by the following two methods.

### (1) Measurement of D-aminoacylase activity:

In 5 mL of 0.1 mol/L phosphate buffer solution (pH 8) were dissolved 0.61 mg of 4-aminoantipyrine (manufactured by Nacalai Tesque, Inc., Code: 01907-52), 3.22 mg of N-ethyl-N-(2-hydroxy-3-sulfopropyl)-3-methlaniline, sodium salt, dihydrate (manufactured by Dojindo Laboratories, Code: OC13), 30 units of peroxidase (manufactured by SIGMA, Code: P-6782), and 1 unit of D-amino acid oxidase (manufactured by SIGMA, Code: A-9128) to prepare a chromogenic reagent. 100 µL of this chromogenic reagent and 100 µL of 100 mmol/L N-acetyl-D,L-valine were mixed in a cell of a microplate with 100 µL of the bacterial cell suspension that had been prepared by subjecting the culture solution as described above to centrifugation and resuspension. After allowing the mixture to react at 37°C for 1 hour, absorbance at 555 nm was measured by using a microplate reader. The strains which were confirmed to show color development were selected as bacterial strains having D-aminoacylase activity.

### (2) Selection of D-aminoacylase-producing bacterium by HPLC analysis:

Next, the strains which had been confirmed by the chromogenic process as described above to have a strong activity for the N-acetyl-D,L-valine were subjected to the HPLC analysis as described below.

The bacterial cells that had been centrifuged after the cultivation were mixed with 100 mmol/L N-acetyl-D,L-valine, and 30 µL of the reaction solution was subjected to analysis by HPLC under the conditions including the use of the column: SUMICHIRA1 OA-5000 (5 µm, 4.6 mm diameter x 150 mm); mobile phase: 2 mmol/L copper sulfate : acetonitrile = 90 : 10; temperature: 40°C; flow rate: 0.8 mL/min; and detection: 230 nm to evaluate the decomposition of the N-acetyl-D,L-amino acid and formation of the D-amino acid or the L-amino acid from the peak areas of the elution of the N-acetyl-D-valine and the N-acetyl-L-valine, and the D-valine and the L-valine. It was then confirmed that the strains that had been selected by the chromogenic process show rapid reduction of the N-acetyl-D-valine and increase of the D-valine in an amount corresponding to the reduction of the N-acetyl-D-valine.

Next, their reactivities with various N-acetyl-D,L-amino acids were compared, and the microorganism which produces an enzyme having a high specificity for a D-amino acid and a high reactivity with D-valine (such reactivity being absent in the conventional D-aminoacylase) was selected as a novel D-aminoacylase-producing bacterial strain.

The bacterial strain obtained by the above procedure as described above had bacteriological characteristics as described above, and this strain was designated as Defluvibacter sp. A131-3 strain.

### Example 2: Production of D-aminoacylase

Defluvibacter sp. A131-3 strain was cultivated in 20 L of the medium at pH 8 prepared by adding 0.1% yeast extract powder D-3 (manufactured by Wako Pure Chemical Industries, Ltd., Code: 390-00531), 0.1% polypeprone (manufactured by Wako Pure Chemical Industries, Ltd., Code: 394-00115), and 0.05% sodium chloride to the culture medium used in Example 1. The cultivation took place in an aerated jar fermenter at 30°C agitated at 150 r/min for 27 hours. At the completion of the cultivation, turbidity (ABS 660 nm) was 1.52, and pH was 7.75.

After the cultivation, the bacterial cells were collected by centrifugation in a refrigerated centrifuge (manufactured by Hitachi Koki Co., Ltd.) at 4000 r/min for 60 minutes, and the collected cells were washed with 20 mmol/L Tris-HCl buffer solution (pH 8), and again centrifuged. The thus collected bacterial cells of 112 g were cryopreserved at -80°C.

The cryopreserved bacterial cells were thawed, and suspended in 340 mL of 20 mmol/L Tris-HCl buffer solution (pH 8) which is an amount three times that of the bacterial cells. The suspension was ultrasonicated for 120 minutes in a low temperature chamber (4°C) by using an injection-type sonicator, and the sonicated suspension was centrifuged in a refrigerated high speed centrifuge (manufactured by Hitachi Koki Co., Ltd.) at 8000 r/min and at 4°C for 60 minutes. The supernatant thus obtained (365 mL) was used as a crude enzyme solution.

This bacterial strain produced D-aminoacylase with no addition of N-acetyl-D,L-valine in the culture medium. However, the amount of the enzyme produced would be at least doubled by adding N-acetyl-D,L-valine to the medium.

### Example 3: Purification of D-aminoacylase

The crude enzyme solution was filled in a dialysis tube and dialyzed against 20 mmol/L Tris-HCl buffer solution (pH 8) containing 0.1 mol/L sodium chloride in a low temperature chamber (4°C) for one day with stirring, while replacing the buffer solution several times. After completing the dialysis, the dialyzate was centrifuged at 8000 r/min and at 4°C for 60 minutes on a refrigerated high speed centrifuge (manufactured by Hitachi Koki Co., Ltd.) to obtain a supernatant (342 mL).

One third of the dialyzate was purified as described below. A 114 mL portion of the dialyzate was introduced into TOYOPEARL SuperQ-650M column (manufactured by Toso Corporation) (4.4 cm diameter x 37.5 cm) that had been equilibrated with 20 mmol/L Tris-HCl buffer solution (pH 8) containing 0.1 mol/L sodium chloride for adsorption of the enzyme. Next, the column was washed with 1500 mL of 20 mmol/L Tris-HCl buffer solution (pH 8) containing 0.1 mol/L sodium chloride, and then, enzyme was eluted with 5700 mL of 20 mmol/L Tris-HCl buffer solution (pH 8) containing 0.1 mol/L sodium chloride and 5700 mL of 20 mmol/L Tris-HCl buffer solution (pH 8) containing 0.3 mol/L sodium chloride by linear concentration gradient method. The eluate from the column was collected into 25 mL fractions, and each fraction was evaluated for the amount of protein (absorbance at 280 nm) and the D-aminoacylase activity (see below for the measurement of enzymatic activity) to recover the active fractions.

The D-aminoacylase enzymatic activity of each fraction was evaluated by using the chromogenic reagent prepared by dissolving 0.61 mg of 4-aminoantipyrine (manufactured by Nacalai Tesque, Inc., Code: 01907-52), 3.22 mg of N-ethyl-N-(2-hydroxy-3-sulfopropyl)-3-methlaniline, sodium salt, dihydrate (manufactured by Dojindo Laboratories, Code: OC13), 30 units of peroxidase (manufactured by SIGMA, Code: P-6782), and 1 unit of D-amino acid oxidase (manufactured by SIGMA, Code: A-9128) in 10 mL of 0.1 mol/L phosphate buffer solution (pH 8). A 1 mL reaction solution containing 500 µL of this chromogenic reagent, 100 µL of 100 mmol/L N-acetyl-D,L-valine, 100 µL of the enzyme sample to be evaluated, and 300 µL of 0.1 mol/L phosphate buffer solution (pH 8) was heated to 37°C for 30 minutes, and the value of absorbance was measured at 555 nm by using a spectrophotometer.

The fractions (968 mL) whose D-aminoacylase activity had been confirmed in the chromatography on TOYOPEARL SuperQ-650M were concentrated using Vivaflow 50 ultrafiltration membrane having a molecular weight cut-off of 10000 (manufactured by SARTORIUS K.K.), and the concentrate was dialyzed against 5 mmol/L phosphate buffer solution (pH 7.2).

160 mL of this dialyzate enzyme solution was adsorbed on BIO-GEL HT hydroxyapatite column (2.2 cm diameter x 20 cm) (manufactured by BIO-RAD) which had been equilibrated with 5 mmol/L phosphate buffer solution (pH 7.2).

Next, the column was washed with 350 mL of 5 mmol/L phosphate buffer solution (pH 7.2), and the enzyme was eluted with 750 mL of 5 mmol/L phosphate buffer solution (pH 7.2) and 750 mL of 200 mmol/L phosphate buffer solution (pH 7.2) by linear concentration gradient method. The eluate from the column was collected into 25 mL fractions, and each fraction was evaluated for the amount of protein and the D-aminoacylase activity to recover the active fractions.

The active fractions (280 ml) obtained by hydroxyapatite chromatography using BIO-GEL HT (manufactured by BIO-RAD) were concentrated to 20 mL using Vivaflow 50 ultrafiltration membrane having a molecular weight cut-off of 10000 (manufactured by SARTORIUS K.K.).

The concentrate thus obtained was applied to Superdex 200 pg column (manufactured by Pharmacia Biotech) (2.2 cm diameter x 66 cm) that had been equilibrated with 20 mmol/L Tris-HCl buffer solution (pH 8) containing 0.3 mol/L sodium chloride, and the same buffer solution was allowed to flow at a flow rate of 1.5 mL/minute. The eluate from the column was collected into 10 mL fractions, and each fraction was evaluated for the amount of protein and the D-aminoacylase activity.

A small portion of the fractions whose D-aminoacylase activity had been confirmed was subjected to the analysis by SDS-polyacrylamide gel electrophoresis (SDS-PAGE) to confirm the absence of protein impurity.

The SDS-polyacrylamide gel electrophoresis was conducted by using PAG Mini "Daiichi" 10/20 (manufactured by Daiichi Pure Chemicals Co.,Ltd.) according to the SDS-polyacrylamide gel electrophoresis process instructed by Daiichi Pure Chemicals Co.,Ltd. 50 µL of SDS-sample treating solution (manufactured by Daiichi Pure Chemicals Co.,Ltd.) and 50 µL of the purified fractions were mixed in equal amounts, and the mixture was boiled for 5 minutes.

To PAG Mini "Daiichi" 10/20 gel was applied 20 µL of the sample that had been boiled, and the electrophoresis was conducted at a constant electric current of 40 mA. After staining the gel with Page Blue 83 Stain Reagent (manufactured by Daiichi Pure Chemicals Co.,Ltd.), the protein band which was estimated to be of the target D-aminoacylase was confirmed.

The active fractions whose purity had been confirmed to be high by the specific activity (enzymatic activity in relation to unit amount of protein) and the electrophoresis were collected, and the these fractions were concentrated by Vivaflow 50 ultrafiltration membrane having a molecular weight cut-off of 10000 (manufactured by SARTORIUS K.K.), and then, by Vivapore 10/20 ultrafiltration membrane having a molecular weight cut-off of 7500 (manufactured by SARTORIUS K.K.) to obtain 28 mL of a purified enzyme.

The enzyme purification yield by this purification method was as shown in Table 1.

**Table 1**

| Stage | Liquid volume (mL) | Total protein content (mg) | Total activity (KU) | Specific activity (U/mg) | Activity yield (%) |
|---|---|---|---|---|---|
| Sonicated and centrifuged supernatant | 114 | 4605 | 2707 | 588 | 100 |
| SuperQ-650M | 968 | 47 | 1334 | 28300 | 49 |
| BIO-GEL HT | 280 | 27 | 1125 | 41600 | 42 |
| Superdex 200 | 346 | 25 | 1003 | 40100 | 37 |
| Conc. solution | 28 | 23 | 953 | 41400 | 35 |

### Example 4: Enzymological properties of the purified enzyme

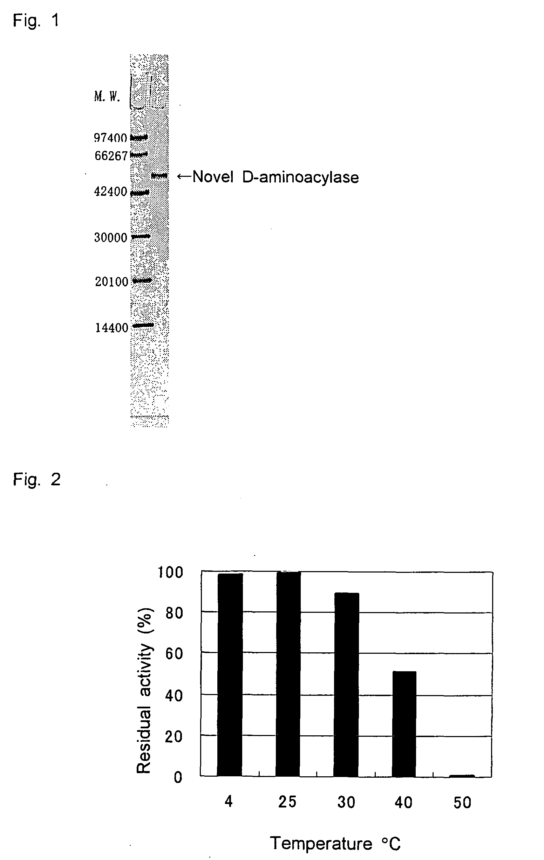

The D-aminoacylase from the Defluvibacter sp. A131-3 strain obtained in Example 3 (hereinafter sometimes referred to as the enzyme of the present invention) was evaluated for its enzymological characteristics by the methods as described below.
1. Molecular weight was measured by the SDS-polyacrylamide gel electrophoresis as described above (using PAG Mini "Daiichi" 10/20 manufactured by Daiichi Pure Chemicals Co.,Ltd.). The molecular weight determined from the mobility of protein molecular weight markers (protein molecular weight markers "Daiichi"·III manufactured by Daiichi Pure Chemicals Co., Ltd.) including phosphorylase b (97,400 daltons), bovine serum albumin (66,267 daltons), aldolase (42,400 daltons), carbonic anhydrase (30,000 daltons), trypsin inhibitor (20,100 daltons), and lysozyme 14,400 daltons) was about 55,000 daltons (FIG. 1).
2. Measurement of the molecular weight by gel filtration was conducted by using Superdex 200pg HR 10/30 (manufactured by Pharmacia Biotech) (1 cm diameter x 30 cm) with 20 mmol/L Tris-HCl buffer solution (pH 8) containing 0.3 mol/L sodium chloride at a flow rate of 1 mL/min, and detecting at 280 nm. The molecular weight markers used were those of LMW Gel Filtration Calibration Kit (manufactured by Pharmacia Biotec) including bovine serum albumin (67,000 daltons), ovalubumin (43,000 daltons), chymotrypsinogen A (25,000 daltons), and ribonuclease A (13,700 daltons), and the relation between the molecular weight and the elution time was determined. The molecular weight obtained by analyzing and calculating the enzyme of the present invention under the same conditions was about 56,000 daltons.
3. Protein isoelectric point was measured on the bases of two-dimensional electrophoresis process by two-dimensional electrophoresis for denatured system (using IPG tube gel "Daiichi" 4-10 and PAG Large "Daiichi" 2D-10/20 manufactured by Daiichi Pure Chemicals Co.,Ltd.). The pI value of the enzyme of the present invention calculated from the mobility of the pI values 5.1, 5.2, 5.3, 5.4, 5.7, 6.0, 6.2, 6.4, 6.5, 6.7, 6.8, 7.0, and 7.1 of the 2D-protein isoelectric point markers (2D-protein isoelectric point markers "Daiichi" manufactured by Daiichi Pure Chemicals Co.,Ltd.) was 5.3.
4. Substrate specificity was determined by an activity measurement method using the chromogenic reagents for the D- and L-amino acid oxidases as described above. More specifically, first calibration curves for the enzymatic activity was depicted by performing reactions using each of the D-amino acids and the L-amino acids at 200 µmol/L, 150 µmol/L, 100 µmol/L, 50 µmol/L, 20 µmol/L, and 10 µmol/L as a substrate, and plotting the absorbance at 555 nm in relation to the substrate concentration.
Next, by using the similar chromogenic reagents with 100 mmol/L N-acetyl-D,L-valine, N-acetyl-D,L-methionine, N-acetyl-D,L-tryptophan, N-acetyl-D,L-leucine, N-acetyl-D,L-phenylalanine, N-acetyl-D,L-tyrosine, and N-acetyl-D,L-glutamic acid as a substrate, the reaction with each acetylamino acid was determined by the procedure as described below.
In 10 mL of 0.1 mol/L phosphate buffer solution (pH 8) were dissolved 0.61 mg of 4-aminoantipyrine (manufactured by Nacalai Tesque, Inc., Code: 01907-52), 3.22 mg of N-ethyl-N-(2-hydroxy-3-sulfopropyl)-3-methlaniline, sodium salt, dihydrate (manufactured by Dojindo Laboratories, Code: OC13), 30 units of peroxidase (manufactured by SIGMA Corp., Code: P-6782), and 1 unit of D-amino acid oxidase (manufactured by SIGMA Corp., Code: A-9128) or 1 unit of L-amino acid oxidase (manufactured by SIGMA Corp., Code: A-5147) to prepare a chromogenic reagent, and 1 mL of a reaction solution containing 500 µL of this chromogenic reagent and 100 µL of each of the acetylamino acid substrate solution at a concentration of 100 mmol/L, 100 µL of the solution of the enzyme of the present invention at a predetermined concentration, and 300 µL of 0.1 mol/L phosphate buffer solution (pH 8) was heated to 37°C for 30 minutes. Absorbance at 555 nm was then measured by a spectrophotometer, and enzymatic activity was determined from the calibration curve that had been prepared by using each of the D-and L-amino acids.
It is to be noted that 1 U corresponds to this amount of the enzyme that catalyzes generation of 1 µmol/L of each D-amino acid or L-amino acid in 1 minute, and the amount of the enzyme was calculated from the relation that had been determined in relation to the concentration of the D-amino acid or the L-amino acid.
The substrate specificity is shown in Table 2 in terms of the relative activity in relation to the activity of N-acetyl-D-methionine and the activity of N-acetyl-D-valine, which are respectively 100.

**Table 2**

| Substrate | Relative activity (% in relation to Met) | Relative activity (% in relation to Val) |
|---|---|---|
| N-Ac-D-Val | 762 | 100 |
| N-Ac-D-Met | 100 | 13 |
| N-Ac-D-Trp | 1.1 | 0.2 |
| N-Ac-D-Leu | 555 | 73 |
| N-Ac-D-Phe | 37 | 4.8 |
| N-Ac-D-Tyr | 8.4 | 1.1 |
| N-Ac-D-Glu | 0 | 0 |
| N-Ac-L-Val | 0 | 0 |
| N-Ac-L-Met | 0 | 0 |
| N-Ac-L-Trp | 0 | 0 |
| N-Ac-L-Leu | 0 | 0 |
| N-Ac-L-Phe | 0 | 0 |
| N-Ac-L-Tyr | 0 | 0 |
| N-Ac-L-Glu | 0 | 0 |

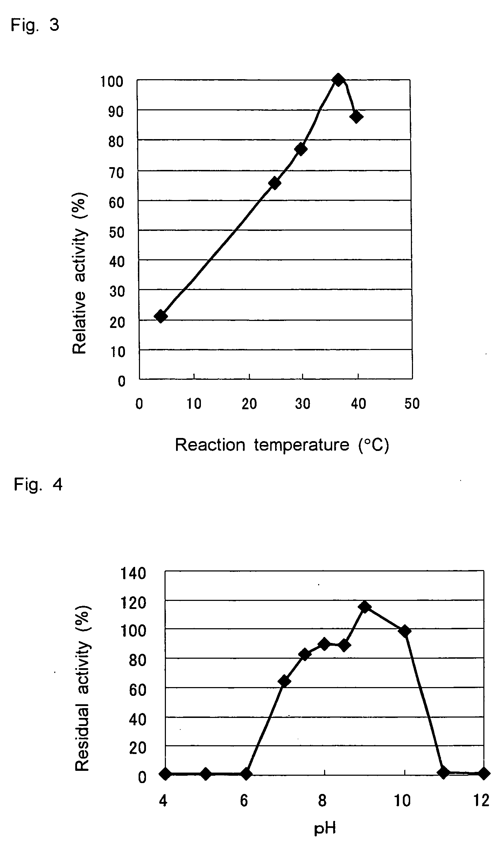

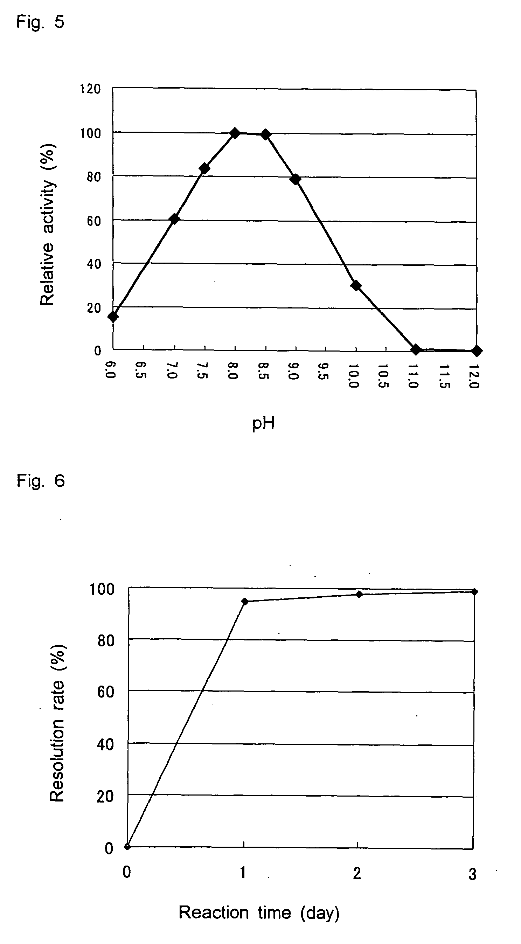

When the reaction was confirmed by using N-acetyl-D-amino acids or N-acetyl-L-amino acids, the enzyme of the present invention acted only on the N-acetyl-D-amino acids and not on the N-acetyl-L-amino acids at all. Of the N-acetyl-D-amino acids, the enzyme of the present invention acted most effectively on N-acetyl-D-valine, and to some extent on N-acetyl-D-leucine, N-acetyl-D-methionine, N-acetyl-D-tryptophan, N-acetyl-D-phenylalanine, and N-acetyl-D-tyrosine, but not on N-acetyl-D-glutamic acid. With regard to the N-acetyl-L-amino acids, the enzyme of the present invention act on none of N-acetyl-L-valine, N-acetyl-L-leucine, N-acetyl-L-methionine, N-acetyl-L-tryptophan, N-acetyl-L-phenylalanine, N-acetyl-L-tyrosine, and N-acetyl-L-glutamic acid.
5. Thermostability was confirmed by heating the solution of the enzyme of the present invention to 4°C, 25°C, 30°C, 40°C, and 50°C for 1 day at pH 8.5 and measuring the activity remaining after the heating by the method of D-aminoacylase activity measurement using the D-amino acid oxidase as described above. The thermostability of the enzyme of the present invention is shown in FIG. 2. The enzyme of the present invention showed residual activity of 80% or more at 4°C to 30°C, demonstrating its stability.
6. Optimal temperature was determined by measuring the activity of the solution of the enzyme of the present invention at pH 8 and at 4°C, 25°C, 30°C, 37°C, and 40°C by the method of D-aminoacylase activity measurement using the D-amino acid oxidase as described above. The optimal temperature of the enzyme of the present invention is shown in FIG. 3. The enzyme of the present invention showed optimal action at 37°C.
7. pH stability was confirmed by heating the enzyme of the present invention to a temperature of 30°C for 1 day at a pH in the range of 4 to 12 and measuring the enzymatic activity remaining after the pH treatment by the method of D-aminoacylase activity measurement using the D-amino acid oxidase as described above. The pH stability of the enzyme of the present invention is shown in FIG. 4. The results indicate that the enzyme of the present invention was most stable near pH 9, and relatively stable with the residual activity of 50% or more near pH 7 to near pH 10. It is to be noted that the residual activity was not 0% at either pH 6 or pH 11.
8. Optimal pH was confirmed by measuring the enzymatic activity of the enzyme of the present invention at a temperature of 37°C and at a pH of 6 to 12 by the method of D-aminoacylase activity measurement using the D-amino acid oxidase as described above. The optimal pH of the enzyme of the present invention is shown in FIG. 5. The enzyme of the present invention was optimally active near pH 8 to pH 8.5.
9. Effects of metal ions were evaluated by adding calcium chloride dihydrate, iron (III) chloride hexahydrate, sodium chloride, cobalt (II) chloride hexahydrate, potassium chloride, nickel chloride hexahydrate, magnesium chloride hexahydrate, copper (II) sulfate pentahydrate, manganese (II) chloride tetrahydrate, zinc chloride, or sodium molybdate to the reaction solution containing 0.5 mol/L N-acetyl-D-valine and the solution of the enzyme of the present invention (500 U) to a final concentration of 1 mmol/L; heating the solution to 40°C for 1 day; measuring the amount of D-valine produced by the HPLC process as described below; and determining the resolution rate for each case of the metal addition in terms of the ratio of the area of D-valine to the area of N-acetyl-D-valine detected and calculating the relative value in relation to the resolution rate of 100% in the case of no metal addition.
As shown in Table 3, activity of the enzyme of the present invention was inhibited by 1 mmol/L Mn²⁺, Co²⁺, Ni²⁺, and Zn²⁺ to the level of relative activity of less than 50%.
HPLC measurement was carried out by using Inertsil ODS-2 column (manufactured by GL Science Inc.) and a buffer solution containing 0.015% 1-sodium pentanesulfonate (pH 2.5) and acetonitrile in a ratio of 80 : 20 at a flow rate of 0.5 mL/minute, and the detection was conducted at 230 nm, and at a column temperature of 30°C.

**Table 3**

| Metal ion, 1.0 mmol/L | Relative activity (%) |
|---|---|
| Not added | 100 |
| Calcium chloride | 99 |
| Iron (III) chloride | 88 |
| Sodium chloride | 99 |
| Cobalt (II) chloride | 27 |
| Potassium chloride | 92 |
| Nickel chloride | 20 |
| Magnesium chloride | 90 |
| Copper (II) sulfate | 90 |
| Manganese chloride | 48 |
| Zinc chloride | 31 |
| Sodium molybdate | 105 |

10. Effects of inhibitor were evaluated by adding ethylenediamine tetraacetic acid, 2-mercaptoethanol, N-ethylmaleimide, o-phenanthroline, L-cysteine, iodacetamide, or dithiothreitol to the reaction solution containing 0.5 mol/L N-acetyl-D-valine and the solution of the enzyme of the present invention (500 U) to a final concentration of 5 mmol/L; heating the solution to 40°C for 1 day; measuring the amount of D-valine produced by the HPLC process as described above; and determining the resolution rate for each case of the inhibitor addition in terms of the ratio of the area of D-valine to the area of N-acetyl-D-valine detected and calculating the relative value in relation to the resolution rate of 100% in the case of no addition of inhibitor.

As shown in Table 4, activity of the enzyme of the present invention was inhibited by 5.0 mmol/L each of dithiothreitol and 2-mercaptoethanol to the relative activity of less than 50%, by 5.0 mmol/L o-phenanthroline to the relative activity of less than 60%, and by 5.0 mmol/L L-cysteine to the relative activity of less than 80%.

**Table 4**

| Inhibitor, 5 mmol/L | Relative activity (%) |
|---|---|
| Ethylenediamine tetraacetic acid | 112 |
| 2-mercaptoethanol | 47 |
| N-ethylmaleimide | 100 |
| O-phenanthroline | 53 |
| L-cysteine | 78 |
| Iodacetamide | 107 |
| Dithiothreitol | 38 |

### Example 5: Cloning of novel D-aminoacylase

The D-aminoacylase purified and isolated from the Defluvibacter sp. A131-3 was analyzed by the method known in the art for the N-terminal and the internal amino acid sequences to thereby obtain the N-terminal sequence (KSFDLVIRNGRVVDP) and the internal sequences (AQAQGLXITXEA and TALIPAQIVER). The primer mix of the N-terminal and the internal sequences including all DNA sequences that are expectable from these amino acids, namely, two primers of ATHMGIAAYGGIMGIGTIGT (SEQ ID NO. 3) and CKYTCIACDATYTGIGCIGGDAT (SEQ ID NO. 4) were prepared. It is to be noted that "I" represents inosine.

Next, genomic DNA was extracted from bacterial cell of the cultivated Defluvibacter sp. A131-3 by a known method.

The primer mix obtained from the purified enzyme and the genomic DNA obtained from the cultivated bacterial cell were used for the PCR using Hot Star Taq (manufactured by QIAGEN). The reaction solution (10 µL) used in the PCR contained in a buffer solution supplied by a manufacturer, 200 µM dNTP, 50 pmol of each primer, 100 ng of the genomic DNA of the Defluvibacter sp. A131-3, and 1 unit of DNA polymerase. The reaction was conducted by denaturing at 95°C for 15 minutes, and then repeating 30 cycles of 1) denaturing at 95°C for 30 seconds; 2) annealing at 40°C for 30 seconds; and 3) synthesis at 72°C for 90 seconds. Agarose electrophoresis confirmed the amplification of the DNA of about 1.3 kb. The PCR product thus obtained was cloned by using pCR2.1topo (manufactured by Invitrogen Corp.) to determine partial base sequence (partial gene).

From the thus obtained partial gene, two primers ATACCGCTACATCGGCAATCGCAT (SEQ ID NO. 5) and TGCCACTGGTTGAAGCCATCGCCA (SEQ ID NO. 6) were designed and synthesized, and PCR reaction was conducted on them by inverse PCR using Hot Star Taq (manufactured by QIAGEN). The template used in the inverse PCR was the one prepared by digesting 5 µg of the genome extracted from Defluvibacter sp. A131-3 with the restriction enzyme SalI (NEB) overnight at 37°C followed by purification and cyclization with T4 Ligase (NEB). The reaction included denaturing at 95°C for 15 minutes followed by 30 cycles of 1) denaturing at 94°C for 30 seconds; 2) annealing at 60°C for 30 seconds; and 3) synthesis at 72°C for 4 minutes. The resulting PCR product was cloned using pCR2.1topo to determine the entire base sequence (entire gene).

Based on the results of this base sequence, primers of exterior of the N-terminal and C-terminal, namely, ATGGCCAAAAGCTTCGATCTC (SEQ ID NO. 7) and TCATCGCGGCGTGCTCCGGATG (SEQ ID NO. 8) were prepared, and a PCR was conducted by using Hot Star Taq (manufactured by QIAGEN) and KOD plus (manufactured by TOYUBO) polymerases. The reaction of the Hot Star Taq was conducted by denaturing at 95°C for 15 minutes, and then, repeating 30 cycles of 1) denaturing at 94°C for 30 seconds; 2) annealing at 58°C for 30 seconds; and 3) synthesis at 72°C for 2 minutes; and the reaction of the KOD plus was conducted by denaturing at 95°C for 2 minutes, and then repeating 30 cycles of 1) denaturing at 94°C for 30 seconds; 2) annealing at 58°C for 30 seconds; and 3) synthesis at 68°C for 2 minutes. The resulting PCR product was cloned by using pCR2.1topo, and the base sequences of the clones were compared to thereby confirm the base sequence of the D-aminoacylase gene of the present invention (SEQ ID NO. 1) and the amino acid sequence of the D-aminoacylase of the present invention (SEQ ID NO. 2).

### Example 6: Production of D-valine

By using 15% aqueous solution of N-acetyl-D,L-valine as a substrate, D-aminoacylase from Defluvibacter sp. A131-3 strain was added with in an amount of 200 U per 1 ml of the aqueous solution of the substrate, and the reaction was allowed to proceed at 40°C for 3 days. The rate of D-valine production by resolution from the N-acetyl-D,L-valine was measured by the HPLC method described in Example 1(2). The results are shown in FIG. 6.

In the system containing the enzyme of the present invention at 200 U/mL in the aqueous solution of the substrate, at least 90% of the N-acetyl-D-valine in the N-acetyl-D,L-valine was converted to D-valine in the first day, and the rate of resolution was at least 90%.

On the other hand, N-acetyl-L-valine was not decomposed at all, indicating the practical utility of the enzyme of the present invention in the production of D-amino acids.

## Claims

1. A D-aminoacylase having the following enzymological properties:
(a) action: acting on a N-acetyl-D-amino acid to produce a D-amino acid;
(b) molecular weight: about 55,000 daltons when determined by SDS-polyacrylamide gel electrophoresis;
(c) isoelectric point: an isoelectric point of 5.3 when measured by two-dimensional electrophoresis for denatured system;
(d) substrate specificity: acting on N-acetyl-D-amino acids, and in particular on N-acetyl-D-valine, but not on N-acetyl-L-amino acids;
acting on substrates such as N-acetyl-D-valine, N-acetyl-D-leucine, N-acetyl-D-methionine, N-acetyl-D-tryptophan, N-acetyl-D-phenylalanine, and N-acetyl-D-tyrosine, but not on N-acetyl-L-valine, N-acetyl-L-leucine, N-acetyl-L-methionine, N-acetyl-L-tryptophan, N-acetyl-L-phenylalanine, and N-acetyl-L-tyrosine;
(e) thermostability: relatively stable at 4°C to 30°C when heated at pH 8.5 for 1 day;
(f) optimal temperature: optimally active at 37°C when reacted at pH 8 for 30 minutes;
(g) pH stability: stable near pH 9, and relatively stable near pH 7 to 10 when heated at a temperature of 30°C for 1 day;
(h) optimal pH: optimally active near pH 8 to 8.5 when reacted at 37°C;
(i) effects of metal ions: activity is inhibited by Mn²⁺, Co²⁺, Ni²⁺, and Zn²⁺ each at 1 mmol/L; and
(j) effects of inhibitors: activity is inhibited by dithiothreitol, 2-mercaptoethanol, o-phenanthroline, and L-cysteine each at 5 mmol/L.

2. A D-aminoacylase comprising a protein defined in either one of (a) and (b):
(a) a protein comprising the amino acid sequence of SEQ ID NO.2; and
(b) a protein comprising an amino acid sequence wherein substitution, deletion, or addition of one to several amino acids has occurred in the amino acid sequence of SEQ ID NO.2, and having D-aminoacylase activity.

3. A gene coding a D-aminoacylase comprising a protein defined in either one of (a) and (b):
(a) a protein comprising the amino acid sequence of SEQ ID NO.2; and
(b) a protein comprising an amino acid sequence wherein substitution, deletion, or addition of one to several amino acids has occurred in the amino acid sequence of SEQ ID NO.2, said protein having D-aminoacylase activity.

4. The gene according to claim 3 comprising a DNA defined in either one of (c) and (d):
(c) a DNA comprising the base sequence of SEQ ID NO. 1; and
(d) a DNA which hybridizes under stringent conditions with the DNA comprising the base sequence which is complimentary to the base sequence of SEQ ID NO. 1, and which codes for a protein having D-aminoacylase activity.

5. A microorganism of genus Defluvibacter which produces a D-aminoacylase that produces a D-amino acid from a N-acetyl-D,L-amino acid or a N-acetyl-D-amino acid.

6. The microorganism according to claim 5 which has been designated Defluvibacter sp. A131-3 and deposited as FERM BP-08563.

7. The microorganism according to claim 5 or 6 which produces the D-aminoacylase of claim 1 or 2.

8. A method for producing the D-aminoacylase of claim 1 or 2 wherein the microorganism of any one of claims 5 to 7 is cultivated, and the D-aminoacylase is recovered from the culture.

9. A method for producing a D-amino acid wherein the D-aminoacylase of claim 1 or 2 is acted on a N-acetyl-D,L-amino acid or a N-acetyl-D-amino acid.


SEQUENCE LISTING

<110> DAIICHI PURE CHEMICALS CO., LTD.
Isobe, Kimiyasu

<120> D-aminoacylase

<130> DC0050

<150> JP 2002-366389
<151> 2002-12-18
<150> JP2003-351560
<151> 2003-10-10

<160> 8

<170> PatentIn version 3.1

<210> 1
<211> 1500
<212> DNA
<213> Defluvibacter sp. A131-3

<400> 1
atggccaaaa gcttcgatct cgtcattcgc aacggcaggg tcgtcgatcc ggaaaccggt 60

catgatgcga ttgccgatgt agcggtatcc ggcggccaga tcgttgcagt cggtccgtcg 120

ctaggtgccg gaaagaggga gatcgacgcg accgggctcg ttgtctcacc gggcttcatt 180

gacctccatg cccacgggca atccattccc gccgaccgga tgcaggcctt cgacggcgtc 240

accaccgcgc tggagcttga ggtgggctcg ctgcccgtcg cgcgctggta cgaacagcag 300

caggccgggg gccgcgtgct caactacggg accgccgctg catggatctt cgcgcgcaag 360

gccgtgatga tcggaatgga actcgatggc cgcctcgcgc cgatcgagat gatgggtgcc 420

ggctccgacg acatgcgctg gtcggtggac gccgcgactg cgccgcagac cgatgatatt 480

gtccggctga cgcgtcaggc tctcgaagaa ggcgcactcg gcatcggcat acctcacggc 540

tatgccgccg gcgctggcgt caaggaaatg acgcgaatct gcgaactggc tgcagaattc 600

gaccggccga cctataccca cattccctac atgtccaaca ttgaccccag aagctcggtc 660

gaggcttatg tgcaactgat cggcctggcc ggtgcaaccg gcgcacacat gcatatctgc 720

caccttaaca gcaccagcct gcgggacgtc gaggatgccg cgaggctgat cgccaaagca 780

caggcacagg gtcttccgat caccaccgag gcctatccct acggcacggg atcgaccgtg 840

atgagcgccc gcttcttcat tgactccgat tttgccgaac gaaccggaac gggctacgac 900

gccatccagg tcgtctcgag cggcaagcgc tttgagaacc gggacgaact cgtggcagcg 960

cgcgccgaaa ccccggaagc actggtgctg tggcattatc tcgacaccga caatccccac 1020

gatcagcggc tgctcgacgt ctcggtgatg tatccgggcg gcgccatcgc ctccgatgcg 1080

gtgccgtgga gcaatcccga cgggacgctg tacaccggcg aggaatggcc gctcccggcc 1140

gacaagacgt cccatccgcg ctcggccggc acctataccc gcttcctcgc ccagtgggtg 1200

cgcgaacgcg aggcggtgcc actggttgaa gccatcgcca aatgcgcgct cattccagcg 1260

cagatcgtcg agcgctgcag cgacgtgttc cgccgcaagg gccggcttca gcccggatgc 1320

gacgccgaca tcgtgatttt cgaccttgaa tccgtgcagg acaggtcaac gttcgaggac 1380

atgcacctcg ccgccgacgg catggtccat gtgctggtca acggcgaggc cgtgatcgcg 1440

aatggcgaac tcgtgcgcga cgcgcgttcc ggccgtgcca tccggagcac gccgcgatga 1500

<210> 2
<211> 499
<212> PRT
<213> Defluvibacter sp.A131-3

<400> 2

Met Ala Lys Ser Phe Asp Leu Val Ile Arg Asn Gly Arg Val Val Asp
1               5                   10                  15

Pro Glu Thr Gly His Asp Ala Ile Ala Asp Val Ala Val Ser Gly Gly
            20                  25                  30

Gln Ile Val Ala Val Gly Pro Ser Leu Gly Ala Gly Lys Arg Glu Ile
        35                  40                  45

Asp Ala Thr Gly Leu Val Val Ser Pro Gly Phe Ile Asp Leu His Ala
    50                  55                  60

His Gly Gln Ser Ile Pro Ala Asp Arg Met Gln Ala Phe Asp Gly Val
65                  70                  75                  80

Thr Thr Ala Leu Glu Leu Glu Val Gly Ser Leu Pro Val Ala Arg Trp
                85                  90                  95

Tyr Glu Gln Gln Gln Ala Gly Gly Arg Val Leu Asn Tyr Gly Thr Ala
            100                 105                 110

Ala Ala Trp Ile Phe Ala Arg Lys Ala Val Met Ile Gly Met Glu Leu
        115                 120                 125

Asp Gly Arg Leu Ala Pro Ile Glu Met Met Gly Ala Gly Ser Asp Asp
    130                 135                 140

Met Arg Trp Ser Val Asp Ala Ala Thr Ala Pro Gln Thr Asp Asp Ile
145                 150                 155                 160

Val Arg Leu Thr Arg Gln Ala Leu Glu Glu Gly Ala Leu Gly Ile Gly
                165                 170                 175

Ile Pro His Gly Tyr Ala Ala Gly Ala Gly Val Lys Glu Met Thr Arg
            180                 185                 190

Ile Cys Glu Leu Ala Ala Glu Phe Asp Arg Pro Thr Tyr Thr His Ile
        195                 200                 205

Pro Tyr Met Ser Asn Ile Asp Pro Arg Ser Ser Val Glu Ala Tyr Val
    210                 215                 220

Gln Leu Ile Gly Leu Ala Gly Ala Thr Gly Ala His Met His Ile Cys
225                 230                 235                 240

His Leu Asn Ser Thr Ser Leu Arg Asp Val Glu Asp Ala Ala Arg Leu
                245                 250                 255

Ile Ala Lys Ala Gln Ala Gln Gly Leu Pro Ile Thr Thr Glu Ala Tyr
            260                 265                 270

Pro Tyr Gly Thr Gly Ser Thr Val Met Ser Ala Arg Phe Phe Ile Asp
        275                 280                 285

Ser Asp Phe Ala Glu Arg Thr Gly Thr Gly Tyr Asp Ala Ile Gln Val
    290                 295                 300

Val Ser Ser Gly Lys Arg Phe Glu Asn Arg Asp Glu Leu Val Ala Ala
305                 310                 315                 320

Arg Ala Glu Thr Pro Glu Ala Leu Val Leu Trp His Tyr Leu Asp Thr
                325                 330                 335

Asp Asn Pro His Asp Gln Arg Leu Leu Asp Val Ser Val Met Tyr Pro
            340                 345                 350

Gly Gly Ala Ile Ala Ser Asp Ala Val Pro Trp Ser Asn Pro Asp Gly
        355                 360                 365

Thr Leu Tyr Thr Gly Glu Glu Trp Pro Leu Pro Ala Asp Lys Thr Ser
    370                 375                 380

His Pro Arg Ser Ala Gly Thr Tyr Thr Arg Phe Leu Ala Gln Trp Val
385                 390                 395                 400

Arg Glu Arg Glu Ala Val Pro Leu Val Glu Ala Ile Ala Lys Cys Ala
                405                 410                 415

Leu Ile Pro Ala Gln Ile Val Glu Arg Cys Ser Asp Val Phe Arg Arg
            420                 425                 430

Lys Gly Arg Leu Gln Pro Gly Cys Asp Ala Asp Ile Val Ile Phe Asp
        435                 440                 445

Leu Glu Ser Val Gln Asp Arg Ser Thr Phe Glu Asp Met His Leu Ala
    450                 455                 460

Ala Asp Gly Met Val His Val Leu Val Asn Gly Glu Ala Val Ile Ala
465                 470                 475                 480

Asn Gly Glu Leu Val Arg Asp Ala Arg Ser Gly Arg Ala Ile Arg Ser
                485                 490                 495

Thr Pro Arg

<210> 3
<211> 20
<212> DNA
<213> artificial sequence

<220>
<223> Designed primer based on Defulvibacter sp.

<220>
<221> misc_feature
<222> (6)..(6)
<223> n stands for i

<220>
<221> misc_feature
<222> (12)..(12)
<223> n stands for i

<220>
<221> misc_feature
<222> (15)..(15)
<223> n stands for i

<220>
<221> misc_feature
<222> (18)..(18)
<223> n stands for i

<400> 3
athmgnaayg gnmgngtngt 20

<210> 4
<211> 23
<212> DNA
<213> artificial sequence

<220>

<223>  Designed primer based on Defluvibacter sp.

<220>
<221>  misc_feature
<222>  (6)..(6)
<223>  n stands for i

<220>
<221>  misc_feature
<222>  (15)..(15)
<223>  n stands for i

<220>
<221>  misc_feature
<222>  (18)..(18)
<223>  n stands for i

<400>  4
ckytcnacda tytgngcngg dat                                              23

<210>  5
<211>  24
<212>  DNA
<213>  artificial sequence

<220>
<223>  Designed primer based on Defluvibacter sp.

<400>  5
ataccgctac atcggcaatc gcat                                             24

<210>  6
<211>  24
<212>  DNA
<213>  artificial sequence

<220>

<223>  Designed primer based on Defluvibacter sp.

<400>  6
tgccactggt tgaagccatc gcca                                              24

<210>  7
<211>  21
<212>  DNA
<213>  artificial sequence

<220>
<223>  Designed primer based on Defluvibacter sp.

<400>  7
atggccaaaa gcttcgatct c                                                 21

<210>  8
<211>  22
<212>  DNA
<213>  artificial sequence

<220>
<223>  Designed primer based on Defluvibacter sp.

<400>  8
tcatcgcggc gtgctccgga tg                                                22